# EUROPEAN PATENT SPECIFICATION

(11) **EP 3 995 082 B1**
(45) Date of publication and mention of the grant of the patent: **10.12.2025**
(21) Application number: 21203913.5
(22) Date of filing: 21.10.2021
(51) Int. Cl.: A61B 6/10, A61B 6/00

(54) **RADIATION IMAGING SYSTEM AND RADIATION IMAGING METHOD**
STRAHLUNGSBILDGEBUNGSSYSTEM UND STRAHLUNGSBILDGEBUNGSVERFAHREN
SYSTÈME ET PROCÉDÉ D'IMAGERIE PAR RAYONNEMENT

(30) Priority: 06.11.2020 US 202063110371 P; 27.08.2021 TW 110131887
(43) Date of publication of application: 11.05.2022
(73) Proprietor: NanoRay Biotech Co., Ltd., Taipei City 11494 (TW)
(72) Inventor: CHENG, Wen-Yuan, Taipei City 114 (TW)
(74) Representative: Becker, Eberhard

(56) References cited:
- EP-A1- 2 517 629
- CN-A- 108 742 671
- US-A1- 2013 343 519
- US-A1- 2017 238 892
- US-A1- 2020 015 702

## Description

### BACKGROUND

### Technical Field

The invention relates to an imaging technology, and in particular to a radiation imaging system according to claim 1 and a radiation imaging method according to claim 10.

### Description of Related Art

X-ray images are a common diagnostic tool in modern radiology. However, due to the danger of X-ray radiation, an X-ray image must be taken in a workplace that meets specific specifications. As a result, the shooting of X-ray images is limited to the site and further requires a certain cost to construct and maintain the safety of the workplace. In addition, a traditional X-ray imaging system has disadvantages including the apparatus being bulky and the system requiring a lot of manpower to set up.

Patent literature CN 108742671 A proposes a hand bone age imaging equipment with an imaging module and an X-ray shielding function, in which the hand bone age imaging equipment includes a rack, an X-ray generator, an X-ray detector, a shielding device and an imaging module, wherein an imaging area is arranged between the X-ray generator and the X-ray detector; the X-ray detector is used for receiving X-rays passing through detected human tissue to generate images; the imaging module is used for acquiring positions and posture states of the detected human tissue and used for transmitting images to an outer image display module for display; the detected human tissue extends into a part above the imaging area from an inspection opening; the X-ray detector is used for receiving the X-rays to generate images.

Patent literature EP 2517629 A1 relates to patient imaging systems, in which the patient imaging system includes an X-ray source emitting X-rays and a wireless X-ray detector detecting the emitted X-rays and acquiring patient image data. The acquisition control system of the patient imaging system initializes and prepares the patient imaging system for X-ray emission and detection; receives the acquired patient image data from the X-ray detector, and non-deterministically controls the operation of the X-ray source and the wireless X-ray detector. The user interfaces of the patient imaging system instruct the acquisition control system when a user is ready for the patient imaging system to initialize and to prepare for X-ray emission and detection and to begin X-ray emission and detection.

Patent literature US 2013/343519 A1 relates to methods for X-ray bone density measurement and imaging for radiography, fluoroscopy and related procedures, peripheral bone density measurement and/or high resolution imaging and/or small field digital radiography of bone and other tissue, including tissue in the peripheral skeletal system, such as the arm, forearm, leg, hand and/or foot.

Patent literature US 2017/238892 A1 relates to a radiation image capturing system including an input unit used to input that a captured radiation image is a capturing failure, a storage storing irradiating amount information regarding capturing set as the capturing failure when there is input that the captured radiation image is the capturing failure, and an output unit that outputs the irradiating amount information regarding the capturing set as the capturing failure stored in the storage.

Patent literature US 2020/015702 A1 relates to a system for landmarking a patient during a medical procedure, such as Magnetic Resonance (MR) Imaging, in which a video display monitor displays images from a video camera positioned in a location relative to an isocenter of the medical device, a patient's position is adjusted to align a feature of interest on the patient with a reference marker displayed on the monitor, and a landmark is declared on the feature of interest such that the system can move the patient to place the landmarked feature of interest substantially at the isocenter of the medical device.

### SUMMARY

The disclosure provides a radiation imaging system and a radiation imaging method, which easily and quickly obtain a radiation image of an object under test.

A radiation imaging system of the present invention includes a remote-control module and an imaging device. The imaging device has a radiation isolation cavity. The radiation isolation cavity includes a radiation irradiation area adapted for placing an object under test. The imaging device includes a controller, a radiation source, and a flat panel detector. The controller is coupled to the remote-control module. The radiation source is coupled to the controller and is disposed on a top of the radiation isolation cavity. The radiation source faces the radiation irradiation area. The flat panel detector is coupled to the controller and is disposed below the radiation irradiation area. During a preparation for exposure, the controller turns on the radiation source. When the controller receives an activation signal output by the remote-control module, the controller operates the flat panel detector to obtain a radiation image corresponding to the object under test.

A radiation imaging method of the present invention uses a radiation imaging system according to claim 1. The radiation imaging method includes the following. During a preparation for exposure, a controller turns on a radiation source disposed on a top of the radiation isolation cavity. During the preparation for exposure, the remote-control module outputs an activation signal. When the controller receives the activation signal, the controller operates a flat panel detector disposed below the radiation irradiation area to obtain a radiation image corresponding to the object under test.

Based on the above, in the radiation imaging system and the radiation imaging method of the present invention, the radiation source irradiates the object under test placed on the radiation irradiation area in the radiation isolation cavity, and the flat panel detector obtains the radiation image corresponding to the object under test. The radiation imaging system and the radiation imaging method of the present invention may realize convenient and effective imaging functions of a radiation image.

To provide a further understanding of the above features and advantages of the invention, embodiments accompanied with drawings are described below in details.

### BRIEF DESCRIPTION OF THE DRAWINGS

FIG. 1 is a schematic circuit diagram of a radiation imaging system according to an embodiment of the disclosure.
FIG. 2 is a flow chart of a radiation imaging method according to an embodiment of the disclosure.
FIG. 3 is a schematic circuit diagram of a radiation imaging system according to another embodiment of the disclosure.
FIG. 4 is a structural side view of an imaging device according to an embodiment of the disclosure.
FIG. 5 is a schematic view of a radiation imaging system according to another embodiment of the disclosure.
FIG. 6 is a flow chart of a radiation imaging method according to another embodiment of the disclosure.
FIG. 7A is a schematic view of a login interface according to an embodiment of the disclosure.
FIG. 7B is a schematic view of a diagnostic object information interface according to an embodiment of the disclosure.
FIG. 7C is a schematic view of an operation interface according to an embodiment of the disclosure.
FIG. 7D is a schematic view of another operation interface according to an embodiment of the disclosure.
FIG. 7E is a schematic view of an operation interface according to yet another embodiment of the disclosure.
FIG. 7F is a schematic view of an image processing interface according to an embodiment of the disclosure.

### DESCRIPTION OF THE EMBODIMENTS

To provide a further understanding of the content of the disclosure, embodiments as examples of how this disclosure may be implemented are described below. In addition, wherever possible, elements/components/steps with the same reference numeral in the drawings and embodiments represent the same or similar components.

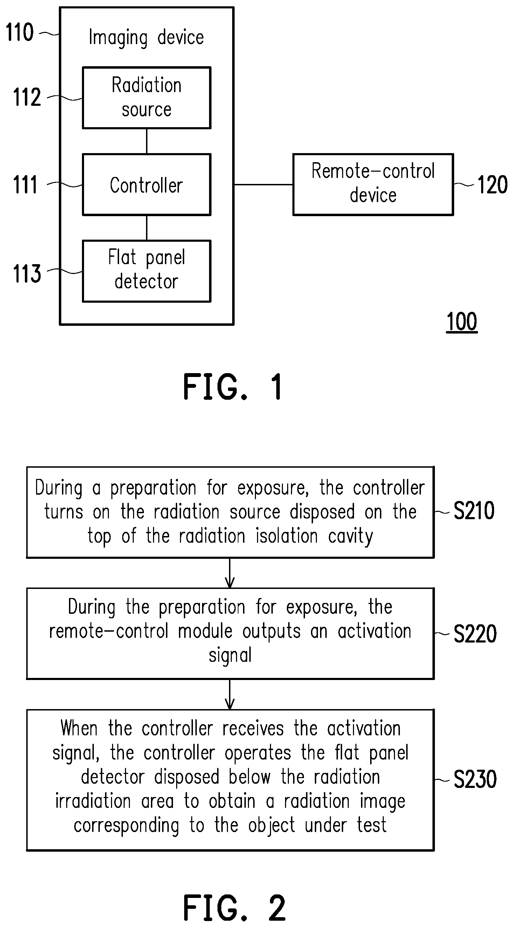

FIG. 1 is a schematic circuit diagram of a radiation imaging system according to an embodiment of the disclosure. Referring to FIG. 1, a radiation imaging system 100 includes an imaging device 110 and a remote-control device 120. The imaging device 110 is coupled to the remote-control device 120. The imaging device 110 may be electrically connected to the remote-control device 120 in a wired or wireless manner. In this embodiment, the imaging device 110 may have a radiation isolation cavity. The radiation isolation cavity includes a radiation irradiation area adapted for placing an object under test. In this embodiment, the imaging device 110 includes a controller 111, a radiation source 112, and a flat panel detector (FPD) 113. The controller 111 is coupled to the remote-control module 120, the radiation source 112 and the flat panel detector 113. The radiation source 112 may be disposed on a top of the radiation isolation cavity. The radiation source 112 may face the radiation irradiation area. The flat panel detector 113 is disposed below the radiation irradiation area. In this embodiment, the radiation source 112 may be an X-ray light source, and the flat panel detector 113 may be an X-ray flat panel sensor. The flat panel detector 113 may generate an X-ray radiation image with a large image range.

In this embodiment, the controller 111 may include a central processing unit (CPU), a microprocessor, a digital signal processor (DSP), a programmable controller, a programmable logic device programmable logic device (PLD) or other similar devices or combinations of these devices, and the disclosure is not limited thereto. The radiation imaging system 100 may further include a storage device, such as a memory. The storage device may be used to store a related control software, operate commands, image data, etc., but the disclosure is not limited thereto. The controller 111 is coupled to the storage device, and may access the related data stored in the storage device, so as to implement the related operations described in each embodiment of the disclosure.

FIG. 2 is a flow chart of a radiation imaging method according to an embodiment of the disclosure. Referring to FIGS. 1 and 2, the radiation imaging system 100 of this embodiment may execute the following steps S210 to S230. In this embodiment, the subject may place the object under test in the radiation irradiation area in the radiation isolation cavity, and the object under test may be, for example, the subject's hand (which may include the palm and/or wrist). Next, in step S210, during a preparation for exposure, the controller 111 turns on the radiation source 112 disposed on the top of the radiation isolation cavity. In step S220, during the preparation for exposure, the remote-control module 120 outputs an activation signal. In step S230, when the controller 111 receives the activation signal, the controller 111 may operate the flat panel detector 113 disposed below the radiation irradiation area to obtain a radiation image corresponding to the object under test. Therefore, the radiation imaging system and the radiation imaging method of the disclosure may realize convenient and effective radiation image imaging functions.

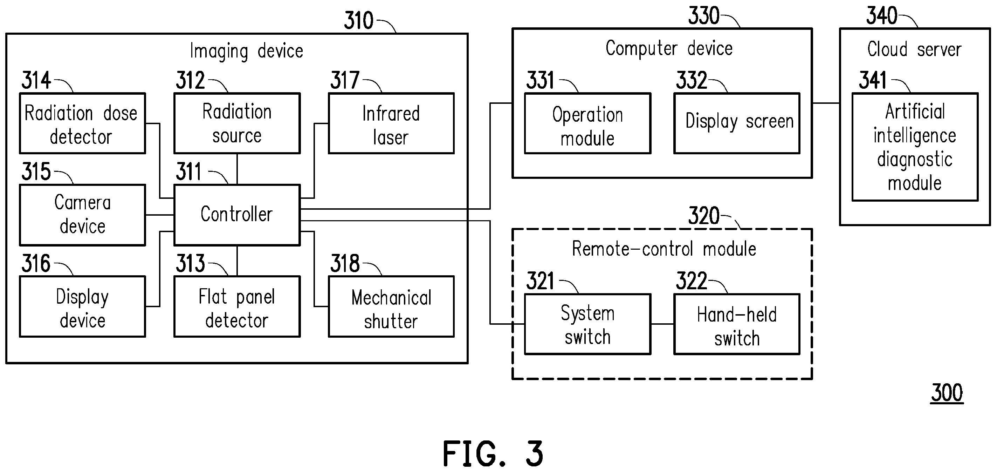

FIG. 3 is a schematic circuit diagram of a radiation imaging system according to another embodiment of the disclosure. Referring to FIG. 3, a radiation imaging system 300 includes an imaging device 310, a remote-control module 320, a computer device 330, and a cloud server 340. In this embodiment, the imaging device 310 includes a controller 311, a radiation source 312, a flat panel detector 313, a radiation dose detector 314, a camera device 315, a display device 316, an infrared laser 317, and a mechanical shutter 318. The controller 311 is coupled to the radiation source 312, the flat panel detector 313, the radiation dose detector 314, the camera device 315, the display device 316, the infrared laser 317, and the mechanical shutter 318. In this embodiment, the imaging device 310 is coupled to the remote-control module 320 and the computer device 330. The computer device 330 is coupled to the cloud server 340. The imaging device 310 may be connected to the remote-control module 320 and the computer device 330 in a wired or wireless manner. The imaging device 310 may transmit a radiation image to the computer device 330. The computer device 330 may be connected to the cloud server 340 in a wired or wireless manner to transmit detection data to the cloud server 340.

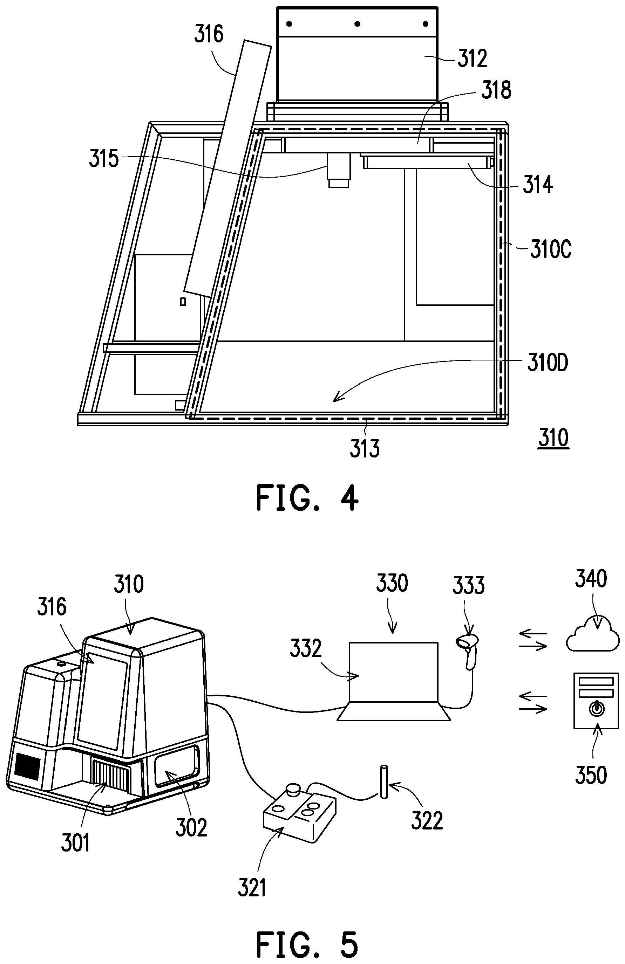

Referring to FIGS. 3 and 4, FIG. 4 is a structural side view of an imaging device according to an embodiment of the disclosure. In this embodiment, the imaging device 310 may be implemented as the structural form shown in FIG. 4, but the disclosure is not limited thereto. In this embodiment, the imaging device 310 has a radiation isolation cavity 310C, and the radiation isolation cavity 310C includes a radiation irradiation area 310D adapted for placing an object under test. As shown in FIG. 4, the radiation source 312, the radiation dose detector 314, the camera device 315, and the mechanical shutter 318 of this embodiment may be disposed on the top of the radiation isolation cavity 310C. According to the invention, the radiation source 312 and the camera device 315 are disposed on top of the radiation isolation cavity 310C. The flat panel detector 313 is disposed below the radiation irradiation area 310D. In addition, the display device 316 is disposed on a case of the imaging device 310 to provide a display function. The radiation source 312, the radiation dose detector 314, and the camera device 315 may face the radiation irradiation area 310D. According to the invention, the camera device 315 and the radiation source 312 face the radiation irradiation area 310D. Furthermore, according to the invention, the radiation isolation cavity 310C is formed by a plurality of lead components (such as lead plates, lead glass, etc.) to provide radiation isolation effects. In addition, the controller 311 of the present embodiment may be disposed at any circuit placement position in the imaging device 310, and the infrared laser 317 may be disposed at any position on the top of the radiation isolation cavity 310C, and the disclosure is not limited thereto.

In this embodiment, the radiation source 312 may be a continuous X-ray light source. The controller 311 may adjust the time length and the light output angle range of an X-ray beam emitted by the radiation source 312 to the radiation irradiation area 310D through operating the mechanical shutter 318. In this embodiment, the radiation dose detector 314 may be used to operate synchronously with the flat panel detector 313. When the flat panel detector 313 obtains the radiation image, the radiation dose detector 314 may obtain the radiation dose information corresponding to the radiation image of a single shot at the same time.

It is worth noting that the imaging device 310 may be a desktop hand diagnostic radiography imaging system, and may be applied, for example, to perform smart children's bone age detection, fracture detection, bone density detection, and gout detection. Therefore, the imaging device 310 of the embodiment may be designed as a desktop detection apparatus with a smaller volume. In this regard, the radiation source 312 and the flat panel detector 313 of this embodiment may have a shorter source image distance (SID) in between; for example, the source image distance may be about 45 centimeters (cm) (a traditional hand X-ray imaging distance is about 1 meter (m)). Therefore, the X-ray beam emitted by the radiation source 312 of the present embodiment during one detection process may have a lower X-ray dose, for example, the X-ray dose may be about 25 uGy (the dose of a traditional hand X-ray is about 50 uGy). In other words, the imaging device 310 of this embodiment may obtain a clear X-ray radiation image of a hand or other objects under test through a lower dose.

In this embodiment, the camera device 315 may be a camera of a charge-coupled device (CCD). The camera device 315 faces the radiation irradiation area 310D, and is used to obtain a video image corresponding to the object under test. In this embodiment, the display device 316 may display the video image. Moreover, in this embodiment, the infrared laser 317 may emit a registration pattern (such as a cross pattern) toward the radiation irradiation area 310D, so that the video image includes the registration pattern. In other words, the imaging device 310 of this embodiment allows the subject to observe the position and posture of the hand placed on the radiation irradiation area 310D in real time through the display device 316, and adjust the position and posture of the hand according to the registration pattern, so that the flat panel detector 313 may capture the correct or appropriate radiation image. In addition, the case of the imaging device 310 of this embodiment may have an emergency stop button disposed thereon.

FIG. 5 is a schematic view of a radiation imaging system according to another embodiment of the disclosure. Referring to FIGS. 3 to 5, in this embodiment, the remote-control module 320 includes a system switch 321 and a hand-held switch 322. As shown in FIG. 5, the remote-control module 320 is disposed outside the imaging device 310. The system switch 321 may be connected to the imaging device 310 in a wired or wireless manner, and the hand-held switch 322 may be coupled to the system switch 321 in a wired manner. In this embodiment, the system switch 321 is used to provide a switching signal to turn on the imaging device 310 through the switching signal. The hand-held switch 322 is used to provide an activation signal. In other words, in the shooting operation of the embodiment, the subject or the operator may turn on and activate the imaging device 310 to execute the imaging operation through operating the remote-control module 320. For example, the subject may be, for example, a child, a patient, or an ordinary person, and the operator may be, for example, a doctor or a nurse. Next, the subject may put his hand into the radiation isolation cavity 310C of the imaging device 310 from a cavity opening 301, and the subject and the operator may view the position and posture of the hand through the display device 316 or a lead glass 302 on a side of the radiation isolation cavity 310C near the radiation irradiation area 310D. In addition, when the subject places the hand correctly in the radiation isolation cavity 310C of the imaging device 310, the operator may remotely operate the imaging device 310 to perform an exposure operation to obtain a radiation image of the subject's hand.

In this embodiment, the computer device 330 may be a related computer apparatus such as a notebook computer or a tablet computer, and the disclosure is not limited thereto. The computer device 330 may include an operation module 331 and a display screen 332. The computer device 330 is used to execute the operation module 331 and displays an operation interface on the display screen 332. In this embodiment, the computer device 330 may further include a processor and a memory, and the memory may pre-store a related program and algorithm of the operation module 331 for the processor to execute. The operation module 331 may be a specific user operation interface and an image processing software. In this embodiment, the imaging device 310 may transmit the video image and the radiation image to the computer device 330. In this regard, the computer device 330 may first display the video image in the operation interface displayed on the display screen 332 during the preparation for exposure. Next, after the flat panel detector 313 obtains the radiation image, the computer device 330 may switch to display the radiation image in the operation interface displayed on the display screen 332. In addition, the operation interface may further include display radiation dose information, whole machine state information, and radiation setting parameter information. In addition, in an embodiment, the computer device 330 may further be coupled to a bar code scanner 333 to, for example, obtain the corresponding diagnostic object information by scanning the bar code on a relevant medical record or body data of the subject. Therefore, the computer device 330 of this embodiment may perform image processing on the radiation image, and perform a profiling operation based on the diagnostic object information and the radiation image to generate a detection file.

In this embodiment, the cloud server 340 may include an artificial intelligence diagnostic module 341. The computer device 330 may, for example, transmit the detection file to the cloud server 340 via the Internet, so that the artificial intelligence diagnostic module 341 of the cloud server 340 may analyze the radiation image and the diagnostic object information in the detection file to automatically generate corresponding diagnostic data. However, as shown in FIG. 5, the computer device 330 may further be connected to a picture archiving and communication system (PACS) 350 to transmit the detection file with the radiation image to the picture archiving and communication system 350 for relevant medical personnel to obtain the detection file through the picture archiving and communication system 350 and perform diagnosis.

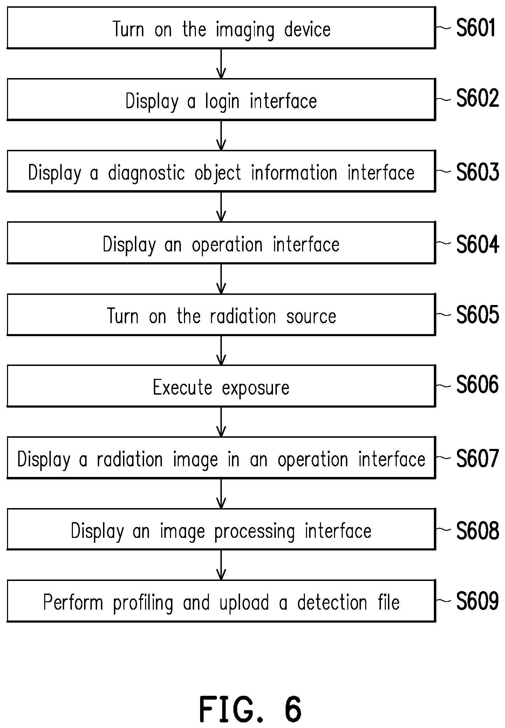

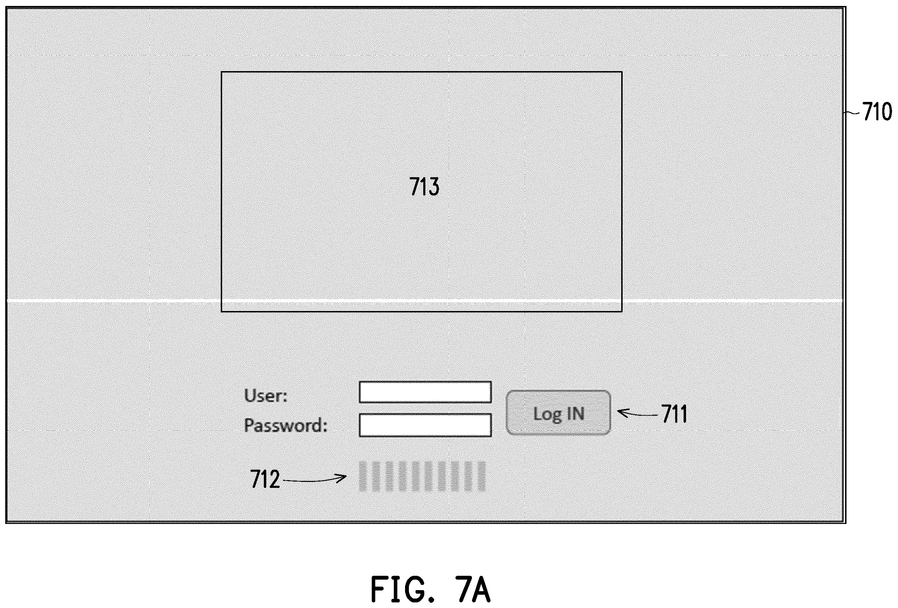

FIG. 6 is a flow chart of a radiation imaging method according to another embodiment of the disclosure. Referring to FIGS. 3 to 6, the radiation imaging system 300 of this embodiment may execute the following steps S601 to S609. In step S601, the operator may turn on the imaging device 310. The imaging device 310 is connected to the computer device 330. Referring to FIG. 7A, FIG. 7A is a schematic view of a login interface according to an embodiment of the disclosure. In step S602, the operation module 331 of the computer device 330 may display a login interface 710 on the display screen 332. As shown in FIG. 7A, the login interface 710 may include a verification area 711, a connection status bar 712, and a welcome screen (or a LOGO screen) 713. In this embodiment, the verification area 711 allows the operator to enter an account and a password to log in to confirm that the operator has sufficient access rights. In this embodiment, the connection status bar 712 may display the booting progress and the connection status of the imaging device 310. In this embodiment, the welcome screen 713 may include a model of the imaging device 310 or the manufacturer's trademark.

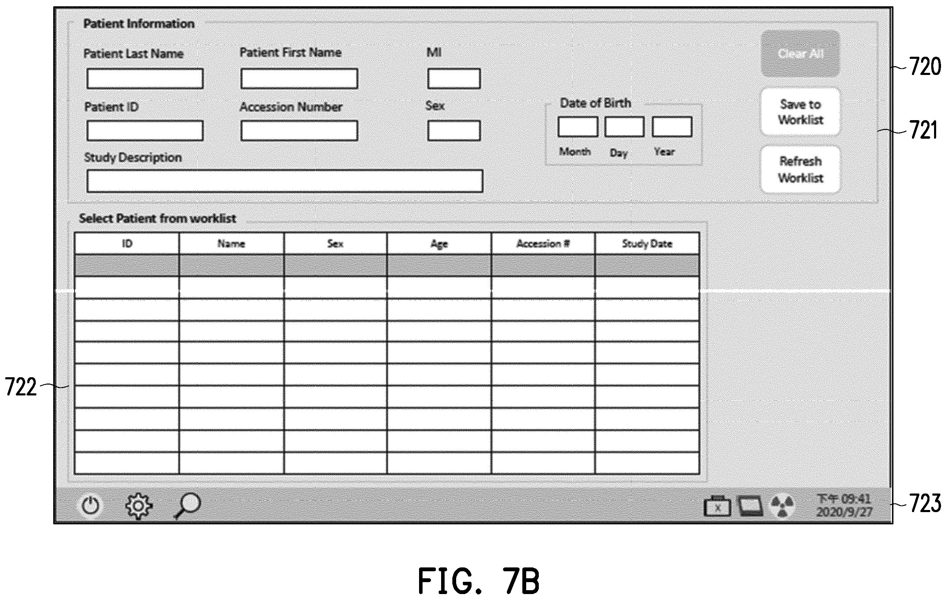

Referring to FIG. 7B, FIG. 7B is a schematic view of a diagnostic object information interface according to an embodiment of the disclosure. In step S603, when the operator performs a login operation through an input apparatus of the computer device 330 to allow the operation module 331 to complete the login operation, the operation module 331 of the computer device 330 may display a diagnostic object information interface 720 on the display screen 332. As shown in FIG. 7B, the diagnostic object information interface 720 may include a data input area 721, a subject list 722, and a status area 723. The data input area 721 may be used to input diagnostic object (subject) information. The diagnosis object information may include a variety of information such as name, date of birth, and notes. In this embodiment, the operator may input relevant data through the input apparatus of the computer device 330, so that the operation module 331 may obtain the diagnostic object information through the diagnostic object information interface 720. Alternatively, in an embodiment, the operator may scan a bar code on a relevant medical record or body data of the subject through the bar code scanner 333 of the operating computer device 330 to obtain the corresponding diagnostic object information.

In this embodiment, the subject list 722 may include a plurality of pieces of diagnostic object information to allow the operator to select and view specific diagnostic object information. The status area 723 may display the status of each element in the current imaging device 310 and the computer device 330. For example, the status area 723 may display the whole machine state information of the radiation source 312 of the imaging device 310, the mechanical shutter 318, the flat panel detector 313, and the display device 316. In addition, the status area 723 may further display the display screen 332 of the computer device 330 and a current network communication connection status.

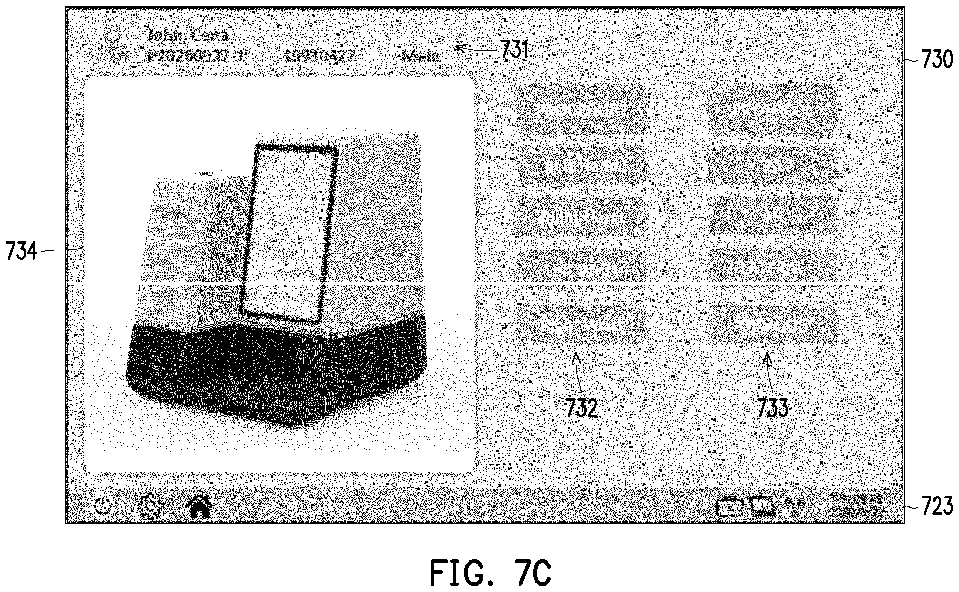

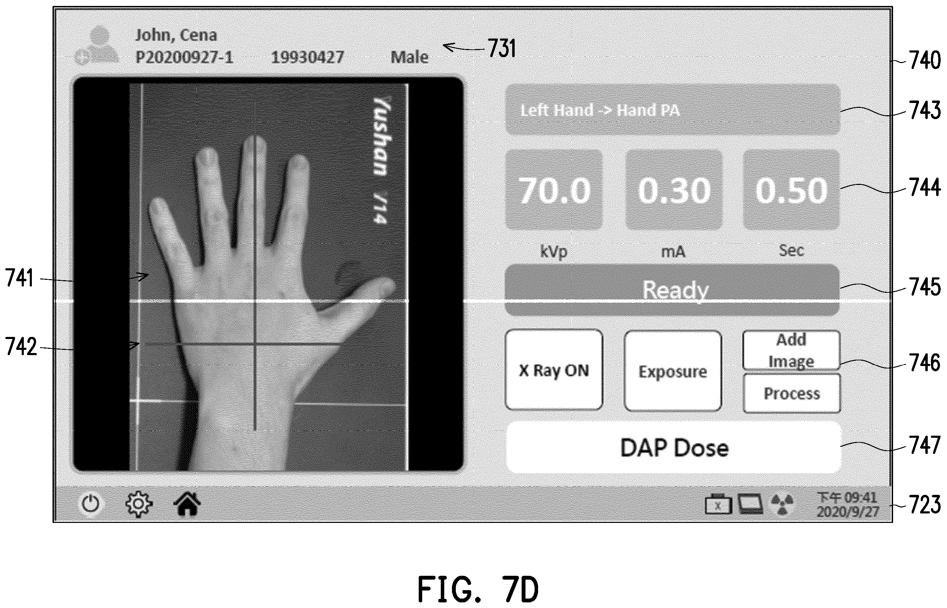

Referring to FIGS. 7C and 7D, FIG. 7C is a schematic view of an operation interface according to an embodiment of the disclosure. FIG. 7D is a schematic view of another operation interface according to an embodiment of the disclosure. In step S604, after the operator completes the input of the diagnostic object information, the operating operation module 331 may display an operation interface 730 on the display screen 332 of the computer device 330. As shown in FIG. 7C, the operation interface 730 may include subject information 731, a shooting part area 732, a shooting positioning area 733, and an example screen 734. The subject information 731 may include the subject information of the current subject. The shooting part area 732 may include, for example, icons of parts available for shooting, such as left hand, right hand, left wrist, and right wrist. The shooting positioning area 733 may, for example, include various icons of positions such as PA, AP, lateral, and oblique. For example, the subject may enter the hand from the cavity opening 301 and place the hand in the radiation irradiation area 310D of the radiation isolation cavity 310C. Next, the subject may select appropriate icons of parts and icons of positions from the shooting part area 732 and the shooting positioning area 733 to record the current posture of the object under test to be shot. In addition, the example screen 734 may display an exemplary screen corresponding to the current part and position. In this embodiment, when the operator completes the selection of the shooting part and shooting position, the operation module 331 switches to display an operation interface 740 as shown in FIG. 7D on the display screen 332 of the computer device 330.

As shown in FIG. 7D, the operation interface 740 may include a video image 741, a registration pattern 742, part and positioning information 743, a radiation setting parameter 744, exposure operational status information 745, a control button area 746, and radiation dose information 747 of an object under test. The video image 741 according to the invention is a real-time image in the radiation isolation cavity 310C captured by the camera device 315. The registration pattern 742 may be a cross pattern projected by the infrared laser 317, but the disclosure is not limited thereto. For example, the operator may view the position and posture of the subject's hand in the radiation irradiation area 310D through the video image 741, and may determine whether the position and posture are correct based on the cross pattern projected on the subject's hand. In this embodiment, the part and positioning information 743 may present the part and position selected by the operator in the previous operation interface 730. The radiation setting parameter 744 may display and adjust the driving parameter of the radiation source 312. The exposure operational status information 745 may display the current operating status of the radiation source 312; for example, the exposure operational status information 745 may indicate that the radiation source 312 is currently in a status ready for exposure. The control button area 746 may be used to select the action to be performed currently. The action may include, for example, turning on the radiation source 312, performing exposure, increasing an exposure image, image processing, etc. The radiation dose information 747 may display the radiation dose detection result provided by the radiation dose detector 314.

In step S605, the operator may operate the control button area 746 of the operation interface 740 to turn on the radiation source 312. It is worth noting that the radiation source 312 enters the preparation for exposure (for example, 10 seconds) to be turned on and provide a continuous X-ray beam. In step S606, during the preparation for exposure, the operator may operate the hand-held switch 322 to execute exposure. In this regard, the mechanical shutter 318 may adjust the X-ray beam of the radiation source 312, and when the sensing pixel of the flat panel detector 313 is exposed, the mechanical shutter 318 may allow the X-ray beam of the radiation source 312 to be emitted to the object under test in the radiation irradiation area at the same time, and allow at least part of the beam to pass through the object under test, so that the flat panel detector 313 may obtain the radiation image of the object under test.

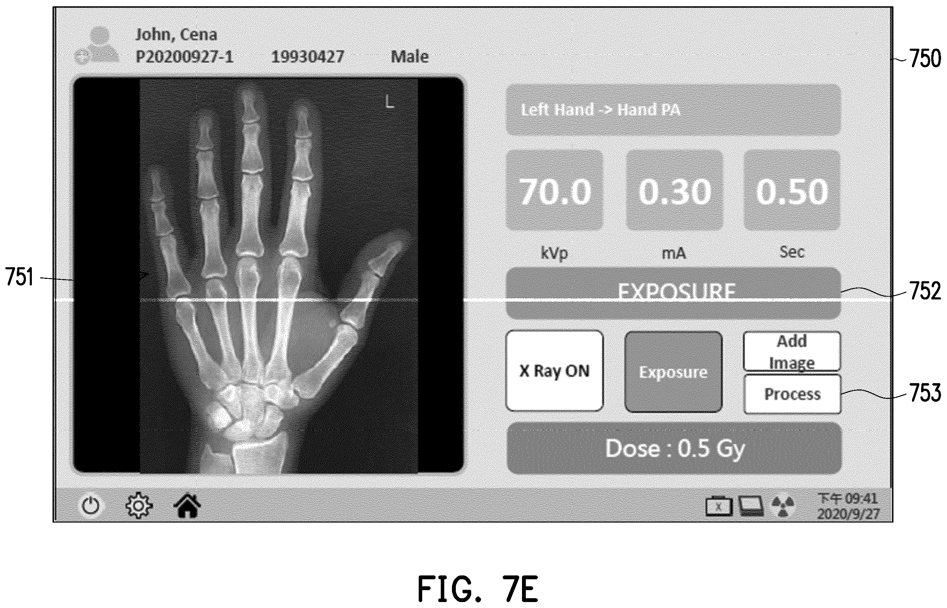

Next, the imaging device 310 may transmit the radiation image to the computer device 330. Referring to FIG. 7E, FIG. 7E is a schematic view of an operation interface according to yet another embodiment of the disclosure. In step S607, the computer device 330 may display a radiation image 751 in an operation interface 750. As shown in FIG. 7E, the operation interface 750 may include the radiation image 751, exposure operational status information 752, and a control button area 753. The radiation image 751 may display an image that has just been captured. The exposure operational status information 752 may display a current operating status of the radiation source 312; for example, the exposure operational status information 752 may indicate that the radiation source 312 is currently in the exposed status. The control button area 753 may be used to display the action that has just been completed or to select the action currently to be performed.

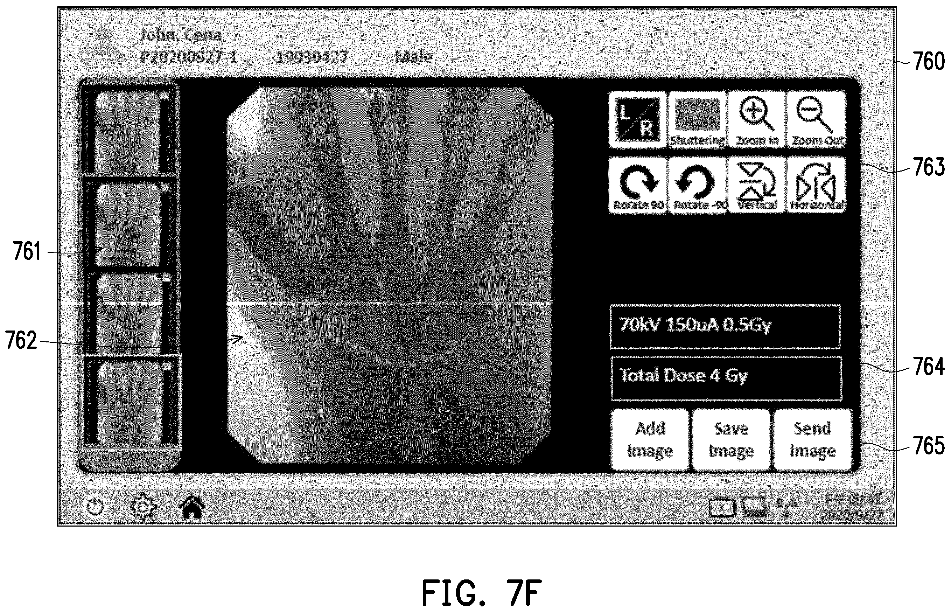

Referring to FIG. 7F, FIG. 7F is a schematic view of an image processing interface according to an embodiment of the disclosure. In step S608, the operator may operate the control button area 753 of the operation interface 750 to perform image processing, so that the operation module 331 may display an image processing interface 760 on the display screen 332. As shown in FIG. 7F, the image processing interface 760 may include an image list 761, a radiation image 762, an adjusting tool area 763, shot information area 764, and a control button area 765. The image list 761 may display a plurality of radiation images that have just been captured. It is worth noting that the radiation images refer to a plurality of images captured by the flat panel detector 313 during a plurality of periods of preparation for exposure. The flat panel detector 313 only captures one shot during one period of preparation for exposure to obtain one radiation image. In this embodiment, the radiation image 762 may be a specific radiation image selected from the image list 761 by the operator. The adjusting tool area 763 may include buttons for various image processing functions such as zooming in, zooming out, rotating, and cropping. The shot information area 764 may include shooting parameter information and radiation dose information corresponding to the selected specific radiation image. The control button area 765 may be used to select the action to be currently performed. The action may include increasing an image, saving an image, and transmitting an image, etc.

In this embodiment, the operator may operate the adjusting tool area 763 of the operation interface 750 to modify the radiation image 762. After the operator finishes modifying the radiation image 762, the operator may operate the control button area 765 of the operation interface 750 to store and transmit the image. In step S609, the operation module 331 may perform profiling and upload a detection file. In this embodiment, the operation module 331 may perform profiling based on the diagnostic object information and the radiation image 760 to generate the detection file. In addition, the computer device 330 may transmit the detection file to the cloud server 340, so that the artificial intelligence diagnostic module 341 of the cloud server 340 analyzes the detection file to automatically generate corresponding diagnostic data.

Therefore, through the radiation imaging system 300 and the radiation imaging method of the disclosure, a radiation image may be easily and quickly obtained, and after the computer device 330 performs real-time image processing on the radiation image, the radiation image may be uploaded to the artificial intelligence diagnostic module 341 of the cloud server 340 for image identification and analysis to automatically generate corresponding diagnostic data.

In summary, through the radiation imaging system and the radiation imaging method of the disclosure, an imaging device may be operated through a computer device so as to obtain a radiation image, and after image optimization processing is performed on the radiation image through the computer device, the radiation image after image optimization may be uploaded to the cloud system for automatic image analysis and diagnosis, so as to generate corresponding diagnostic data. The imaging device of the disclosure has the characteristics of small size and low radiation dose, so the imaging device may be applied to various detection environments and detection objects. In addition, the radiation imaging system of the disclosure has the advantages of being able to be set up quickly and having low requirements for the detection environment and apparatus.

## Claims

1. A radiation imaging system (100, 300), comprising:
a remote-control module (120, 320); and
an imaging device (110, 310), having a radiation isolation cavity (310C), wherein the radiation isolation cavity (310C) comprises a radiation irradiation area (310D) adapted for placing an object under test and the radiation isolation cavity is formed by lead components, the lead components comprising lead plates or lead glass, wherein
the imaging device (110, 310) comprises:
a controller (111, 311), coupled to the remote-control module (120, 320);
a radiation source (112, 312), coupled to the controller (111, 311), disposed on a top of the radiation isolation cavity (310C), wherein the radiation source (112, 312) faces the radiation irradiation area (310D);
a flat panel detector (113, 313), coupled to the controller (111, 311), disposed below the radiation irradiation area (310D);
a camera device (315), coupled to the controller (111, 311), disposed on the top of the radiation isolation cavity (310C), wherein the camera device (315) faces the radiation irradiation area (310D) and is configured to obtain a real-time image (741) corresponding to the object under test; and
a display device (316), coupled to the controller (111,311), configured to display the real-time image (741), wherein the display device (316) is disposed on a case of the imaging device (110, 310),
wherein the controller (111, 311) is configured to turn on the radiation source (112, 312) during a preparation for exposure, and further, the controller (111, 311) is configured to operate the flat panel detector (113, 313) when receiving an activation signal output by the remote-control module (120, 320) to obtain a radiation image (751, 762) corresponding to the object under test.

2. The radiation imaging system (100, 300) according to claim 1, wherein the imaging device (110, 310) further comprises:
a mechanical shutter (318), coupled to the controller (111, 311),
wherein the radiation source (112, 312) is a continuous X-ray light source, and the controller (111, 311) is configured to operate the mechanical shutter (318) to adjust an X-ray beam emitted by the radiation source (112, 312).

3. The radiation imaging system (100, 300) according to claim 1, wherein the imaging device (110, 310) further comprises:
a radiation dose detector (314), coupled to the controller (111, 311), disposed in the radiation isolation cavity (310C), wherein the radiation dose detector (314) is configured to operate synchronously with the flat panel detector (113, 313) to obtain radiation dose information corresponding to the radiation image.

4. The radiation imaging system (100, 300) according to claim 1, wherein the remote-control module (120, 320) comprises:
a system switch (321), coupled to the controller (111, 311), configured to provide a switching signal to turn on the imaging device (110, 310) through the switching signal; and
a hand-held switch (322), coupled to the system switch (321), configured to provide the activation signal.

5. The radiation imaging system (100, 300) according to claim 1, wherein the imaging device (110, 310) further comprises:
an infrared laser (317), coupled to the controller (111, 311), disposed on the top of the radiation isolation cavity (310C), wherein the infrared laser (317) is configured to emit a registration pattern (742) toward the radiation irradiation area (310D) so that the real-time image (741) comprises the registration pattern (742).

6. The radiation imaging system (100, 300) according to claim 1, further comprising:
a computer device (330), coupled to the imaging device (110, 310), comprising a display screen (332),
wherein the computer device (330) is configured to execute an operation module (331), the operation module (331) being a user operation interface and an image processing software, and the user operation interface is configured to be displayed on the display screen (332),
wherein the computer device (330) is configured to first display the real-time image (741) in the user operation interface during the preparation for exposure, and after the flat panel detector (113, 313) is configured to obtain the radiation image (751, 762), the computer device (330) is configured to display the radiation image (751, 762) in the user operation interface,
wherein the user operation interface further comprises radiation dose information, whole machine state information, and radiation setting parameter information.

7. The radiation imaging system (100, 300) according to claim 6, wherein a control button area (746, 753) in the user operation interface is configured such that when it is operated, the operation module (331) displays an image processing interface (760) on the display screen (332), the image processing interface (760) comprises the radiation image (751, 762) and a plurality of image processing function buttons, and the operation module (331) is configured to correspondingly modify the radiation image (751, 762) according to an operation result of the image processing function buttons.

8. The radiation imaging system (100, 300) according to claim 6, wherein the operation module (331) is configured to display a login interface (710) on the display screen (332), and when the operation module (331) is configured to complete a login operation, the operation module (331) is configured to display a diagnostic object information interface (720) on the display screen (332),
wherein the diagnostic object information interface (720) comprises a data input area (721), a subject list (722) and a status area (723),
wherein the operation module (331) is configured to:
obtain diagnostic object information through the data input area (721) of the diagnostic object information interface (720);
select specific diagnostic object information through the subject list (722); and
display machine state information of the radiation source (112, 312) through the status area (723).

9. The radiation imaging system (100, 300) according to claim 8, wherein the operation module (331) is configured to generate a detection file according to the diagnostic object information and the radiation image (751, 762), wherein the detection file comprises the diagnostic object information and the radiation image, and the computer device (330) is configured to communicate with a cloud server (340),
wherein the computer device (330) is configured to transmit the detection file to the cloud server (340), so that an artificial intelligence diagnostic module (341) of the cloud server (340) can analyze the detection file.

10. A radiation imaging method using a radiation imaging system (100, 300) according to claim 1, wherein the radiation imaging method comprises:
during a preparation for exposure, turning on the radiation source (112, 312) disposed on a top of the radiation isolation cavity (310C) by the controller (111, 311);
during a preparation for exposure, outputting an activation signal by the remote-control module (120, 320); and
when the controller (111, 311) receives the activation signal, operating the flat panel detector (113, 313 by the controller (111, 311) to obtain a radiation image (751, 762) corresponding to the object under test.

11. The radiation imaging method according to claim 10, wherein the radiation source (112, 312) is a continuous X-ray light source, and the radiation imaging method further comprises:
operating a mechanical shutter (318) by the controller (111, 311) to adjust an X-ray beam emitted by the radiation source (112, 312).

12. The radiation imaging method according to claim 10, further comprising:
operating a radiation dose detector (314) disposed in the radiation isolation cavity (310C) synchronously with the flat panel detector (113, 313) to obtain radiation dose information corresponding to the radiation image (751, 762).

13. The radiation imaging method according to claim 10, further comprising:
providing a switching signal by a system switch (321) of the remote-control module (120, 320) to turn on the imaging device (110, 310) through the switching signal; and
providing the activation signal by a hand-held switch (322) of the remote-control module (120, 320).

14. The radiation imaging method according to claim 10, further comprising:
obtaining a real-time image (741) corresponding to the object under test by the camera device (315) disposed on the top of the radiation isolation cavity (310C); and
displaying the real-time image (741) by a display device (316).

15. The radiation imaging method according to claim 14, further comprising:
emitting a registration pattern (742) toward the radiation irradiation area (310D) by an infrared laser (317) disposed on the top of the radiation isolation cavity (310C), so that the real-time image (741) comprises the registration pattern (742).

16. The radiation imaging method according to claim 14, wherein the radiation imaging system (100, 300) further comprises a computer device (330), and the radiation imaging method further comprises:
executing an operation module (331) by the computer device (330), wherein the operation module (331) is a user operation interface and an image processing software, and displaying the user operation interface on a display screen (332) of the computer device (330);
during the preparation for exposure, displaying the image (741) in the user operation interface by the computer device (330) first; and
after the flat panel detector (113, 313) is configured to obtain the radiation image (751, 762), displaying the radiation image (751, 762) in the user operation interface by the computer device (330);
wherein the user operation interface further comprises radiation dose information, whole machine state information, and radiation setting parameter information.

17. The radiation imaging method according to claim 16, further comprising:
when a control button area (746, 753) in the user operation interface is operated, displaying an image processing interface (760) on the display screen (332) by the operation module (331), wherein the image processing interface (760) comprises the radiation image (751, 762) and a plurality of image processing function buttons; and
correspondingly modifying the radiation image (751, 762) by the operation module (331) according to an operation result of the image processing function buttons.

18. The radiation imaging method according to claim 16, further comprising:
displaying a login interface (710) on the display screen (332) by the operation module (331);
after the operation module (331) completes a login operation, displaying a diagnostic object information interface (720) on the display screen (332) by the operation module (331), wherein the diagnostic object information interface (720) comprises a data input area (721), a subject list (722) and a status area (723);
obtaining diagnostic object information through the data input area (721) of the diagnostic object information interface (720) by the operation module (331);
selecting specific diagnostic object information through the subject list (722); and
displaying machine state information of the radiation source (112, 312) through the status area (723).

19. The radiation imaging method according to claim 18, further comprising:
generating a detection file by the operation module (331) according to the diagnostic object information and the radiation image (751, 762), wherein the detection file comprises the diagnostic object information and the radiation image;
communicating with a cloud server (340) by the computer device (330);
transmitting the detection file to the cloud server (340) by the computer device (330); and
analyzing the detection file by an artificial intelligence diagnostic module (341) of the cloud server (340).

## Patentansprüche

1. Strahlungsbildgebungssystem (100, 300), umfassend:
ein Fernbedienmodul (120, 320); und
eine Bildgebungsvorrichtung (110, 310) mit einer Strahlungsabschirmkammer (310C), wobei die Strahlungsabschirmkammer (310C) einen Bestrahlungsbereich für Strahlung (310D) umfasst, der zum Anordnen eines Prüfobjekts angepasst ist, und die Strahlungsabschirmkammer aus Bleikomponenten ausgebildet ist, wobei die Bleikomponenten Bleiplatten oder Bleiglas umfassen, wobei die Bildgebungsvorrichtung (110, 310) umfasst:
eine Steuereinheit (111, 311), gekoppelt mit dem Fernbedienmodul (120, 320);
eine Strahlungsquelle (112, 312), gekoppelt mit der Steuereinheit (111, 311), angeordnet auf einer Oberseite der Strahlungsabschirmkammer (310C), wobei die Strahlungsquelle (112, 312) dem Bestrahlungsbereich für Strahlung (310D) zugewandt ist;
einen Flachpaneldetektor (113, 313), gekoppelt mit der Steuereinheit (111, 311), angeordnet unterhalb des Bestrahlungsbereichs für Strahlung (310D);
eine Kameravorrichtung (315), gekoppelt mit der Steuereinheit (111, 311), angeordnet auf der Oberseite der Strahlungsabschirmkammer (310C), wobei die Kameravorrichtung (315) dem Bestrahlungsbereich für Strahlung (310D) zugewandt ist und konfiguriert ist, ein Echtzeitbild (741) entsprechend dem Prüfobjekt zu erfassen; und
eine Anzeigevorrichtung (316), gekoppelt mit der Steuereinheit (111, 311), die konfiguriert ist, das Echtzeitbild (741) anzuzeigen, wobei die Anzeigevorrichtung (316) an einem Gehäuse der Bildgebungsvorrichtung (110, 310) angeordnet ist,
wobei die Steuereinheit (111, 311) konfiguriert ist, die Strahlungsquelle (112, 312) während einer Belichtungsvorbereitung einzuschalten, und ferner wobei die Steuereinheit (111, 311) konfiguriert ist, den Flachpaneldetektor (113, 313) zu betreiben, wenn ein von dem Fernbedienmodul (120, 320) ausgegebenes Aktivierungssignal empfangen wird, um ein Strahlungsbild (751, 762) entsprechend dem Prüfobjekt zu erhalten.

2. Strahlungsbildgebungssystem (100, 300) nach Anspruch 1, wobei die Bildgebungsvorrichtung (110, 310) ferner umfasst:
einen mechanischen Verschluss (318), gekoppelt mit der Steuereinheit (111, 311),
wobei die Strahlungsquelle (112, 312) eine kontinuierliche Röntgenquelle ist und die Steuereinheit (111, 311) konfiguriert ist, den mechanischen Verschluss (318) zu betätigen, um einen von der Strahlungsquelle (112, 312) emittierten Röntgenstrahl einzustellen.

3. Strahlungsbildgebungssystem (100, 300) nach Anspruch 1, wobei die Bildgebungsvorrichtung (110, 310) ferner umfasst:
einen Strahlungsdosisdetektor (314), gekoppelt mit der Steuereinheit (111, 311), angeordnet in der Strahlungsabschirmkammer (310C), wobei der Strahlungsdosisdetektor (314) konfiguriert ist, synchron mit dem Flachpaneldetektor (113, 313) zu arbeiten, um Strahlungsdosisinformationen zu erhalten, die dem Strahlungsbild entsprechen.

4. Strahlungsbildgebungssystem (100, 300) nach Anspruch 1, wobei das Fernbedienmodul (120, 320) umfasst:
einen Systemschalter (321), gekoppelt mit der Steuereinheit (111, 311), konfiguriert, ein Schaltsignal bereitzustellen, um die Bildgebungsvorrichtung (110, 310) mittels des Schaltsignals einzuschalten; und
einen Handschalter (322), gekoppelt mit dem Systemschalter (321), konfiguriert, das Aktivierungssignal bereitzustellen.

5. Strahlungsbildgebungssystem (100, 300) nach Anspruch 1, wobei die Bildgebungsvorrichtung (110, 310) ferner umfasst:
einen Infrarotlaser (317), gekoppelt mit der Steuereinheit (111, 311), angeordnet auf der Oberseite der Strahlungsabschirmkammer (310C), wobei der Infrarotlaser (317) konfiguriert ist, ein Registriermuster (742) in Richtung des Bestrahlungsbereichs für Strahlung (310D) zu emittieren, sodass das Echtzeitbild (741) das Registriermuster (742) umfasst.

6. Strahlungsbildgebungssystem (100, 300) nach Anspruch 1, ferner umfassend:
eine Rechnervorrichtung (330), gekoppelt mit der Bildgebungsvorrichtung (110, 310), umfassend einen Anzeigeschirm (332),
wobei die Rechnervorrichtung (330) konfiguriert ist, ein Bedienmodul (331) auszuführen, wobei das Bedienmodul (331) eine Benutzerschnittstelle und eine Bildverarbeitungssoftware ist und die Benutzerschnittstelle auf dem Anzeigeschirm (332) darzustellen ist,
wobei die Rechnervorrichtung (330) konfiguriert ist, während der Belichtungsvorbereitung zunächst das Echtzeitbild (741) in der Benutzerschnittstelle anzuzeigen und, nachdem der Flachpaneldetektor (113, 313) konfiguriert ist, das Strahlungsbild (751, 762) zu erhalten, die Rechnervorrichtung (330) konfiguriert ist, das Strahlungsbild (751, 762) in der Benutzerschnittstelle anzuzeigen,
wobei die Benutzerschnittstelle ferner Strahlungsdosisinformationen, Gesamtgeräte-Statusinformationen und Informationen zu Strahlungseinstellparametern umfasst.

7. Strahlungsbildgebungssystem (100, 300) nach Anspruch 6, wobei ein Steuertastenbereich (746, 753) in der Benutzerschnittstelle so konfiguriert ist, dass bei dessen Betätigung das Bedienmodul (331) eine Bildverarbeitungsschnittstelle (760) auf dem Anzeigeschirm (332) darstellt, wobei die Bildverarbeitungsschnittstelle (760) das Strahlungsbild (751, 762) und eine Vielzahl von Bildverarbeitungs-Funktionstasten umfasst, und das Bedienmodul (331) konfiguriert ist, das Strahlungsbild (751, 762) anhand eines Betätigungsergebnisses der Bildverarbeitungs-Funktionstasten zu verändern.

8. Strahlungsbildgebungssystem (100, 300) nach Anspruch 6, wobei das Bedienmodul (331) konfiguriert ist, eine Anmeldeschnittstelle (710) auf dem Anzeigeschirm (332) darzustellen und, wenn das Bedienmodul (331) konfiguriert ist, einen Anmeldevorgang zu abzuschließen, das Bedienmodul (331) konfiguriert ist, eine Diagnostik-Objekt-Informationsschnittstelle (720) auf dem Anzeigeschirm (332) darzustellen,
wobei die Diagnostik-Objekt-Informationsschnittstelle (720) einen Dateneingabebereich (721), eine Subjektliste (722) und einen Statusbereich (723) umfasst,
wobei das Bedienmodul (331) konfiguriert ist:
Diagnostik-Objektinformationen über den Dateneingabebereich (721) der Diagnostik-Objekt-Informationsschnittstelle (720) zu erhalten;
spezifische Diagnostik-Objektinformationen über die Subjektliste (722) auszuwählen; und
Gerätestatusinformationen der Strahlungsquelle (112, 312) über den Statusbereich (723) anzuzeigen.

9. Strahlungsbildgebungssystem (100, 300) nach Anspruch 8, wobei das Bedienmodul (331) konfiguriert ist, eine Erfassungsdatei anhand der Diagnostik-Objektinformationen und des Strahlungsbildes (751, 762) zu erzeugen, wobei die Erfassungsdatei die Diagnostik-Objektinformationen und das Strahlungsbild umfasst, und die Rechnervorrichtung (330) konfiguriert ist, mit einem Cloud-Server (340) zu kommunizieren,
wobei die Rechnervorrichtung (330) konfiguriert ist, die Erfassungsdatei an den Cloud-Server (340) zu übertragen, sodass ein Künstliche-Intelligenz-Diagnosemodul (341) des Cloud-Servers (340) die Erfassungsdatei analysieren kann.

10. Strahlungsbildgebungsverfahren unter Verwendung eines Strahlungsbildgebungssystems (100, 300) nach Anspruch 1, wobei das Strahlungsbildgebungsverfahren umfasst:
während einer Belichtungsvorbereitung, Einschalten der auf der Oberseite der Strahlungsabschirmkammer (310C) angeordneten Strahlungsquelle (112, 312) durch die Steuereinheit (111, 311);
während einer Belichtungsvorbereitung, Ausgeben eines Aktivierungssignals durch das Fernbedienmodul (120, 320); und
wenn die Steuereinheit (111, 311) das Aktivierungssignal empfängt, Betreiben des Flachpaneldetektors (113, 313) durch die Steuereinheit (111, 311), um ein Strahlungsbild (751, 762) entsprechend dem Prüfobjekt zu erhalten.

11. Strahlungsbildgebungsverfahren nach Anspruch 10, wobei die Strahlungsquelle (112, 312) eine kontinuierliche Röntgenquelle ist, und das Strahlungsbildgebungsverfahren ferner umfasst:
Betätigen eines mechanischen Verschlusses (318) durch die Steuereinheit (111, 311), um einen von der Strahlungsquelle (112, 312) emittierten Röntgenstrahl einzustellen.

12. Strahlungsbildgebungsverfahren nach Anspruch 10, ferner umfassend:
Betreiben eines in der Strahlungsabschirmkammer (310C) angeordneten Strahlungsdosisdetektors (314) synchron mit dem Flachpaneldetektor (113, 313), um Strahlungsdosisinformationen zu erhalten, die dem Strahlungsbild (751, 762) entsprechen.

13. Strahlungsbildgebungsverfahren nach Anspruch 10, ferner umfassend:
Bereitstellen eines Schaltsignals durch einen Systemschalter (321) des Fernbedienmoduls (120, 320), um die Bildgebungsvorrichtung (110, 310) mittels des Schaltsignals einzuschalten; und
Bereitstellen des Aktivierungssignals durch einen Handschalter (322) des Fernbedienmoduls (120, 320).

14. Strahlungsbildgebungsverfahren nach Anspruch 10, ferner umfassend:
Erhalten eines Echtzeitbildes (741) entsprechend dem Prüfobjekt durch die auf der Oberseite der Strahlungsabschirmkammer (310C) angeordnete Kameravorrichtung (315); und
Anzeigen des Echtzeitbildes (741) durch eine Anzeigevorrichtung (316).

15. Strahlungsbildgebungsverfahren nach Anspruch 14, ferner umfassend:
Emittieren eines Registriermusters (742) in Richtung des Bestrahlungsbereichs für Strahlung (310D) durch einen auf der Oberseite der Strahlungsabschirmkammer (310C) angeordneten Infrarotlaser (317), sodass das Echtzeitbild (741) das Registriermuster (742) umfasst.

16. Strahlungsbildgebungsverfahren nach Anspruch 14, wobei das Strahlungsbildgebungssystem (100, 300) ferner eine Rechnervorrichtung (330) umfasst, und wobei das Verfahren ferner umfasst:
Ausführen eines Bedienmoduls (331) durch die Rechnervorrichtung (330), wobei das Bedienmodul (331) eine Benutzerschnittstelle und eine Bildverarbeitungssoftware ist, und Darstellen der Benutzerschnittstelle auf einem Anzeigeschirm (332) der Rechnervorrichtung (330);
während der Belichtungsvorbereitung, anfängliches Anzeigen des Bildes (741) in der Benutzerschnittstelle durch die Rechnervorrichtung (330); und
nachdem der Flachpaneldetektor (113, 313) konfiguriert ist, das Strahlungsbild (751, 762) zu erhalten, Anzeigen des Strahlungsbildes (751, 762) in der Benutzerschnittstelle durch die Rechnervorrichtung (330);
wobei die Benutzerschnittstelle ferner Strahlungsdosisinformationen, Gesamtgeräte-Statusinformationen und Informationen zu Strahlungseinstellparametern umfasst.

17. Strahlungsbildgebungsverfahren nach Anspruch 16, ferner umfassend:
wenn ein Steuertastenbereich (746, 753) in der Benutzerschnittstelle betätigt wird, Darstellen einer Bildverarbeitungsschnittstelle (760) auf dem Anzeigeschirm (332) durch das Bedienmodul (331), wobei die Bildverarbeitungsschnittstelle (760) das Strahlungsbild (751, 762) und eine Vielzahl von Bildverarbeitungs-Funktionstasten umfasst; und
entsprechendes Verändern des Strahlungsbildes (751, 762) durch das Bedienmodul (331) anhand eines Betätigungsergebnisses der Bildverarbeitungs-Funktionstasten.

18. Strahlungsbildgebungsverfahren nach Anspruch 16, ferner umfassend:
Darstellen einer Anmeldeschnittstelle (710) auf dem Anzeigeschirm (332) durch das Bedienmodul (331);
nachdem das Bedienmodul einen Anmeldevorgang abgeschlossen hat, Darstellen einer Diagnostik-Objekt-Informationsschnittstelle (720) auf dem Anzeigeschirm (332), wobei die Diagnostik-Objekt-Informationsschnittstelle (720) einen Dateneingabebereich (721), eine Subjektliste (722) und einen Statusbereich (723) umfasst;
Erhalten von Diagnostik-Objektinformationen über den Dateneingabebereich (721);
Auswählen spezifischer Diagnostik-Objektinformationen über die Subjektliste (722); und
Anzeigen von Gerätestatusinformationen der Strahlungsquelle (112, 312) über den Statusbereich (723).

19. Strahlungsbildgebungsverfahren nach Anspruch 18, ferner umfassend:
Erzeugen einer Erfassungsdatei durch das Bedienmodul (331) anhand der Diagnostik-Objektinformationen und des Strahlungsbildes (751, 762), wobei die Erfassungsdatei die Diagnostik-Objektinformationen und das Strahlungsbild umfasst;
Kommunikation mit einem Cloud-Server (340) durch die Rechnervorrichtung (330);
Übertragen der Erfassungsdatei an den Cloud-Server (340) durch die Rechnervorrichtung (330); und
Analysieren der Erfassungsdatei durch ein Künstliche-Intelligenz-Diagnosemodul (341) des Cloud-Servers (340).

## Revendications

1. Système d'imagerie par rayonnement (100, 300), comprenant:
un module de commande à distance (120, 320); et
un dispositif d'imagerie (110, 310), comportant une cavité d'isolation des rayonnements (310C), dans lequel la cavité d'isolation des rayonnements (310C) comprend une zone d'irradiation des rayonnements (310D) adaptée pour placer un objet à tester et la cavité d'isolation des rayonnements est formée par des composants en plomb, les composants en plomb comprenant des plaques de plomb ou du verre au plomb, dans lequel le dispositif d'imagerie (110, 310) comprend:
un contrôleur (111, 311), couplé au module de commande à distance (120, 320);
une source de rayonnement (112, 312), couplée au contrôleur (111, 311), disposée sur le dessus de la cavité d'isolation contre les rayonnements (310C), dans laquelle la source de rayonnement (112, 312) fait face à la zone d'irradiation par rayonnement (310D);
un détecteur à panneau plat (113, 313), couplé au contrôleur (111, 311), disposé sous la zone d'irradiation (3100); un dispositif de caméra (315), couplé au contrôleur (111, 311), disposé sur le dessus de la cavité d'isolation des rayonnements (310C), dans lequel le dispositif de caméra (315) fait face à la zone d'irradiation des rayonnements (310D) et est configuré pour obtenir une image en temps réel (741) correspondant à l'objet testé; et
un dispositif d'affichage (316), couplé au contrôleur (111, 311), configuré pour afficher l'image en temps réel (741), dans lequel le dispositif d'affichage (316) est disposé sur un boîtier du dispositif d'imagerie (110, 310) dans lequel le contrôleur (111, 311) est configuré pour allumer la source de rayonnement (112, 312) pendant une préparation à l'exposition, et en outre le contrôleur (111, 311) est configuré pour actionner le détecteur à panneau plat (113, 313) lorsqu'il reçoit un signal d'activation émis par le module de commande à distance (120, 320) afin d'obtenir une image de rayonnement (751, 762) correspondant à l'objet testé.

2. Système d'imagerie par rayonnement (100, 300) selon la revendication 1, dans lequel le dispositif d'imagerie (110, 310) comprend en outre :un obturateur mécanique (318), couplé au contrôleur (111, 311), dans lequel la source de rayonnement (112, 312) est une source de lumière à rayons X continue, et le contrôleur (111, 311) est configuré pour actionner l'obturateur mécanique (318) afin de régler un faisceau de rayons X émis par la source de rayonnement (112, 312).

3. Système d'imagerie par rayonnement (100, 300) selon la revendication 1, dans lequel le dispositif d'imagerie (110, 310) comprend en outre:
un détecteur de dose de rayonnement (314), couplé au contrôleur (111, 311), disposé dans la cavité d'isolation des rayonnements (310C), dans lequel le détecteur de dose de rayonnement (314) est configuré pour fonctionner de manière synchrone avec le détecteur à panneau plat (113, 313) afin d'obtenir des informations sur la dose de rayonnement correspondant à l'image de rayonnement.

4. Système d'imagerie par rayonnement (100, 300) selon la revendication 1, dans lequel le module de commande à distance (120, 320) comprend:
un commutateur de système (321), couplé au contrôleur (111, 311), configuré pour fournir un signal de commutation afin d'activer le dispositif d'imagerie (110, 310) par le biais du signal de commutation; et
un commutateur portatif (322), couplé au commutateur de système (321), configuré pour fournir le signal d'activation.

5. Système d'imagerie par rayonnement (100, 300) selon la revendication 1, dans lequel le dispositif d'imagerie (110, 310) comprend en outre :un laser infrarouge (317), couplé au contrôleur (111, 311), disposé sur le dessus de la cavité d'isolation des rayonnements (310C), dans lequel le laser infrarouge (317) est configuré pour émettre un motif d'enregistrement (742) vers la zone d'irradiation des rayonnements (310D) de sorte que l'image en temps réel (741) comprend le motif d'enregistrement (742).

6. Système d'imagerie par rayonnement (100, 300) selon la revendication 1, comprenant en outre:
un dispositif informatique (330), couplé au dispositif d'imagerie (110, 310), comprenant un écran d'affichage (332),dans lequel le dispositif informatique (330) est configuré pour exécuter un module d'opération (331), le module opérationnel (331) étant une interface opérationnelle utilisateur et un logiciel de traitement d'image, et l'interface opérationnelle utilisateur étant configurée pour être affichée sur l'écran d'affichage (332), dans lequel le dispositif informatique (330) est configuré pour afficher d'abord l'image en temps réel (741) dans l'interface opérationnelle utilisateur pendant la préparation de l'exposition, et après que le détecteur à panneau plat (113, 313) est configuré pour obtenir l'image de rayonnement (751, 762), le dispositif informatique (330) est configuré pour afficher l'image de rayonnement (751, 762) dans l'interface utilisateur,
dans lequel l'interface utilisateur comprend en outre des informations sur la dose de rayonnement, des informations sur l'état global de la machine et des informations sur les paramètres de réglage du rayonnement.

7. Système d'imagerie par rayonnement (100, 300) selon la revendication 6, dans lequel une zone de boutons de commande (746, 753) dans l'interface utilisateur est configurée de telle sorte que, lorsqu'elle est actionnée, le module de commande (331) affiche une interface de traitement d'image (760) sur l'écran d'affichage (332), l'interface de traitement d'image (760) comprend l'image de rayonnement (751, 762) et une pluralité de boutons de fonction de traitement d'image, et le module de commande (331) est configuré pour modifier de manière correspondante l'image de rayonnement (751, 762) en fonction d'un résultat de fonctionnement des boutons de fonction de traitement d'image.

8. Système d'imagerie par rayonnement (100, 300) selon la revendication 6, dans lequel le module de commande (331) est configuré pour afficher une interface de connexion (710) sur l'écran d'affichage (332), et lorsque le module de commande (331) est configuré pour effectuer une opération de connexion, le module de commande (331) est configuré pour afficher une interface d'informations sur l'objet diagnostiqué (720) sur l'écran d'affichage (332), dans lequel l'interface d'informations sur l'objet diagnostiqué (720) comprend une zone de saisie de données (721), une liste de sujets (722) et une zone d'état (723),dans lequel le module de fonctionnement (331) est configuré pour : obtenir des informations sur l'objet diagnostiqué via la zone de saisie de données (721) de l'interface d'informations sur l'objet diagnostiqué (720);
sélectionner des informations spécifiques sur l'objet diagnostiqué via la liste des sujets (722); et
afficher des informations sur l'état de la machine de la source de rayonnement (112, 312) via la zone d'état (723).

9. Système d'imagerie par rayonnement (100, 300) selon la revendication 8, dans lequel le module d'opération (331) est configuré pour générer un fichier de détection en fonction des informations sur l'objet diagnostiqué et de l'image de rayonnement (751, 762), dans lequel le fichier de détection comprend les informations sur l'objet diagnostiqué et l'image de rayonnement, et le dispositif informatique (330) est configuré pour communiquer avec un serveur cloud (340),dans lequel le dispositif informatique (330) est configuré pour transmettre le fichier de détection au serveur cloud (340), de sorte qu'un module de diagnostic d'intelligence artificielle (341) du serveur cloud (340) puisse analyser le fichier de détection.

10. Procédé d'imagerie par rayonnement utilisant un système d'imagerie par rayonnement (100, 300) selon la revendication 1, dans lequel le procédé d'imagerie par rayonnement comprend :pendant une préparation à l'exposition, l'activation de la source de rayonnement (112, 312) disposée sur le dessus de la cavité d'isolation des rayonnements (310C) par le contrôleur (111, 311) ;pendant une préparation à l'exposition, la sortie d'un signal d'activation par le module de commande à distance (120, 320); et
lorsque le contrôleur (111, 311) reçoit le signal d'activation, actionner le détecteur à panneau plat (113, 313) par le contrôleur (111, 311) afin d'obtenir une image de rayonnement (751, 762) correspondant à l'objet testé.

11. Procédé d'imagerie par rayonnement selon la revendication 10, dans lequel la source de rayonnement (112, 312) est une source de lumière à rayons X continue, et le procédé d'imagerie par rayonnement comprend en outre:
l'actionnement d'un obturateur mécanique (318) par le contrôleur (111, 311) afin d'ajuster un faisceau de rayons X émis par la source de rayonnement (112, 312).

12. Procédé d'imagerie par rayonnement selon la revendication 10, comprenant en outre:
l'actionnement d'un détecteur de dose de rayonnement (314) disposé dans la cavité d'isolation de rayonnement (310C) de manière synchrone avec le détecteur à panneau plat (113, 313) afin d'obtenir des informations de dose de rayonnement correspondant à l'image de rayonnement (751, 762).

13. Procédé d'imagerie par rayonnement selon la revendication 10, comprenant en outre:
la fourniture d'un signal de commutation par un commutateur système (321) du module de commande à distance (120, 320) pour allumer le dispositif d'imagerie (110, 310) par le biais du signal de commutation; et
la fourniture du signal d'activation par un commutateur portatif (322) du module de commande à distance (120, 320).

14. Procédé d'imagerie par rayonnement selon la revendication 10, comprenant en outre:
l'obtention d'une image en temps réel (741) correspondant à l'objet testé par le dispositif de caméra (315) disposé sur le dessus de la cavité d'isolation des rayonnements (310C); et
l'affichage de l'image en temps réel (741) par un dispositif d'affichage (316).

15. Procédé d'imagerie par rayonnement selon la revendication 14, comprenant en outre:
l'émission d'un motif d'enregistrement (742) vers la zone d'irradiation par rayonnement (310D) par un laser infrarouge (317) disposé sur le dessus de la cavité d'isolation par rayonnement (310C), de sorte que l'image en temps réel (741) comprend le motif d'enregistrement (742).

16. Procédé d'imagerie par rayonnement selon la revendication 14, dans lequel le système d'imagerie par rayonnement (100, 300) comprend en outre un dispositif informatique (330), et le procédé d'imagerie par rayonnement comprend en outre:
l'exécution d'un module opérationnel (331) par le dispositif informatique (330), dans lequel le module opérationnel (331) est une interface utilisateur et un logiciel de traitement d'image, et l'affichage de l'interface utilisateur sur un écran d'affichage (332) du dispositif informatique (330);pendant la préparation de l'exposition, l'affichage de l'image (741) dans l'interface utilisateur par le dispositif informatique (330) en premier;
et après que le détecteur à panneau plat (113, 313) a été configuré pour obtenir l'image de rayonnement (751, 762), l'affichage de l'image de rayonnement (751, 762) dans l'interface utilisateur par le dispositif informatique (330) ;dans lequel l'interface utilisateur comprend en outre des informations sur la dose de rayonnement, des informations sur l'état global de la machine et des informations sur les paramètres de réglage du rayonnement.

17. Procédé d'imagerie par rayonnement selon la revendication 16, comprenant en outre:
lorsqu'une zone de boutons de commande (746, 753) dans l'interface utilisateur est actionnée, l'affichage d'une interface de traitement d'image (760) sur l'écran d'affichage (332) par le module de commande (331), dans lequel l'interface de traitement d'image (760) comprend l'image de rayonnement (751, 762) et une pluralité de boutons de fonction de traitement d'image; et
en modifiant en conséquence l'image de rayonnement (751, 762) par le module de commande (331) en fonction du résultat de l'action sur les boutons de fonction de traitement d'image.

18. Procédé d'imagerie par rayonnement selon la revendication 16, comprenant en outre:
l'affichage d'une interface de connexion (710) sur l'écran d'affichage (332) par le module de commande (331);une fois que le module d'opération (331) a terminé une opération de connexion, l'affichage d'une interface d'informations sur l'objet diagnostiqué (720) sur l'écran d'affichage (332) par le module d'opération (331), dans lequel l'interface d'informations sur l'objet diagnostiqué (720) comprend une zone de saisie de données (721), une liste de sujets (722) et une zone d'état (723) ; obtenir des informations sur l'objet diagnostiqué via la zone de saisie de données (721) de l'interface d'informations sur l'objet diagnostiqué (720) par le module d'opération (331) ;sélectionner des informations spécifiques sur l'objet diagnostiqué via la liste des sujets (722); et
afficher des informations sur l'état de la machine de la source de rayonnement (112, 312) via la zone d'état (723).

19. Procédé d'imagerie par rayonnement selon la revendication 18, comprenant en outre:
la génération d'un fichier de détection par le module d'opération (331) en fonction des informations sur l'objet diagnostiqué et de l'image de rayonnement (751, 762), dans lequel le fichier de détection comprend les informations sur l'objet diagnostiqué et l'image de rayonnement; la communication avec un serveur cloud (340) par le dispositif informatique (330); la transmission du fichier de détection au serveur cloud (340) par le dispositif informatique (330); et
l'analyse du fichier de détection par un module de diagnostic d'intelligence artificielle (341) du serveur cloud (340).
